# FASCICULE DE BREVET EUROPEEN

(11) **EP 2 646 501 B1**
(45) Date de publication et mention de la délivrance du brevet: **26.04.2017**
(21) Numéro de dépôt: 11788186.2
(22) Date de dépôt: 28.11.2011
(51) Int. Cl.: A61K 45/06, A61K 47/46, A61K 31/352, A61K 35/741, A61K 36/48, C08L 89/04, C08J 5/18, A61K 9/00, A61K 9/70

(54) **FILMS NATURELS BIODEGRADABLES A BASE DE CO-PRODUITS ISSUS DE PROCESSUS INDUSTRIELS DE TRAITEMENT DE GRAINES**
NATÜRLICHE UND BIOLOGISCH ABBAUBARE FILME AUF DER BASIS VON NEBENPRODUKTEN AUS DER INDUSTRIELLEN BEHANDLUNG VON KÖRNERN
NATURAL AND BIODEGRADABLE FILMS BASED ON CO-PRODUCTS DERIVED FROM THE INDUSTRIAL TREATMENT OF CORNS

(30) Priorité: 29.11.2010 FR 1059871
(43) Date de publication de la demande: 09.10.2013
(73) Titulaire: NUTRINOV, 35530 Noyal-sur-Vilaine (FR)
(72) Inventeur: EFSTATHIOU, Théo, F-35740 Pace (FR); AUDIC, Jean-Luc, F-35190 Becherel (FR); DIVERS, Thomas, 42000 Saint Etienne (FR)
(74) Mandataire: Vidon Brevets & Stratégie
(86) Numéro de dépôt international: PCT/EP2011/071196
(87) Numéro de publication internationale: WO 2012/072587

(56) Documents cités:
- EP-A1- 1 132 084
- GB-A- 1 510 999
- DATABASE WPI Week 200102 Thomson Scientific, London, GB; AN 2001-011149 XP002642065, & JP 2000 281917 A (IDEMITSU PETROCHEM CO LTD) 10 octobre 2000 (2000-10-10)
- MONEDERO F.M., FABRA M.J., TALENS P., CHIRALT A.: "Effect of calcium and sodium caseinates on physical characteristics of soy protein isolate -lipid films", JOURNAL OF FOOD ENGINEERING, vol. 97, 20 octobre 2009 (2009-10-20), pages 228-234, XP002642066,
- SU J., YUAN X., HUANG Z., XIA W-L: "Properties stability and biodegradation behaviors of soy protein isolate/poly (vinyl alcohol) blend films", POLYMER DEGRADATION AND STABILITY, vol. 95, 14 avril 2010 (2010-04-14), pages 1226-1237, XP002642067,

## Description

L'invention concerne le domaine des matériaux biodégradables et plus précisément celui des films réalisés à partir de produits naturels.

Les problématiques liées à l'écologie et à la protection de l'environnement sont de plus en plus présentes et intégrées au sein des politiques industrielles, notamment dans le secteur de l'industrie chimique. La « chimie propre » ou « chimie verte » tend à réduire, voire à éliminer, l'emploi ou la production de substances dangereuses dans la conception, la fabrication et l'application des produits chimiques.

Trois domaines se détachent :
- la conception de technologies novatrices pour assurer la disponibilité et l'emploi d'énergies renouvelables,
- le développement de ressources renouvelables et des produits qui en dérivent,
- la création de technologies qui n'entraînent pas de pollution.

Une des sources non négligeable de pollution provient de la fabrication et de l'utilisation de films plastiques, notamment les emballages et les sacs plastiques.

Les films plastiques conventionnels, généralement à base de polymères vinyliques, représentent 4 % de la consommation de pétrole (une matière première fossile non renouvelable) et constituent plus de 1 000 KT/an de déchets dont seulement 20 % sont recyclés.

Dans ce contexte, le développement des films biodégradables naturels représente un enjeu majeur.

On connaît le document EP1132084A1 qui décrit un pansement transdermique comprenant un film et une matrice posée sur le film, ainsi qu'une colle non allergisante à partir d'isoflavones de soja. On connaît également le document JP2000-281917-A1qui porte sur des emballages formés à partir de résidus de feuilles de thé ou d'extraction de café. La publication de F. Maria Monedero et al. (Effect of calcium and sodium caseinates on physical characterics of soy protein isolate-lipid films, J. of Food Engineering 97 (2010) :228 :234) décrit les résultats de travaux de recherches portant sur l'effet des caseinates de sodium et calcium sur les caractéristiques physiques de films produits à partir d'isolats protéiques de soja. Des travaux de recherche ont porté sur la stabilité et la biodégradation de films produits à partir de mélange d'isolats protéiques de soja et d'alcool polyvinylique (J.F.Su et al, Properties stability and biodégradation behaviors of soy protein isolate/poly(vinyl alcohol) blend films, Polymer Degradation and Stability, 95 (2010) 1226-1237). On connaît également le document GB1510999 qui décrit un film plastique soluble dans l'eau ou les solvants organiques, et formé d'un matériau choisi parmi les dérivés cellulosiques ou polyvinyliques.

Par ailleurs, plusieurs réalisations industrielles de films à base d'amidon ont été réalisées avec succès. L'amidon est un glucide de réserve produit et utilisé par les végétaux supérieurs pour stocker de l'énergie. On le retrouve dans les organes de réserve des plantes, les graines (en particulier dans les céréales et les légumineuses), les racines, les tubercules et les rhizomes (pomme de terre, patate douce, manioc, etc.). Sur le plan industriel, c'est surtout le maïs et la pomme de terre qui sont utilisés. Outre le fait que l'amidon est une matière première renouvelable, c'est également une matière non toxique et totalement naturelle.

Toutefois, ces films présentent deux inconvénients majeurs:
- leur dégradation reste lente, de l'ordre de quelques mois à quelques années,
- ils continuent à intégrer dans leur composition un pourcentage réduit mais non négligeable de composés non biodégradables qui sont susceptibles de se retrouver dans les sols après dégradation du film et qui s'avèrent très difficiles à récupérer.

L'objectif de la présente invention est de fournir un film naturel et biodégradable ne présentant pas les inconvénients des films de l'état de la technique.

En particulier, un des objectifs de l'invention est de fournir un film naturel et biodégradable n'intégrant pas dans sa composition de composés non biodégradables.

Un autre objectif de l'invention est de proposer un tel film dont la dégradation est améliorée par rapport aux films de l'état de la technique, c'est-à-dire une dégradation beaucoup plus poussée et plus rapide.

Un autre objectif est de proposer un tel film qui soit relativement peu coûteux à fabriquer et qui soit en accord avec les exigences de la « chimie propre ».

Un objectif de la présente invention est également de fournir, dans certains de ses modes de réalisation de celle-ci, des films biodégradables pour une utilisation dans le domaine pharmaceutique ou médical.

L'invention concerne un film biodégradable caractérisé en ce qu'il est réalisé à partir d'au moins un co-produit issu d'un processus de traitement des graines de soja visant à l'obtention de produits finis à base de soja par ultrafiltration, ledit co-produit étant choisi dans le groupe comprenant l'okara et le perméat.

On entend par « co-produits », les produits autres que le produit principal ou les produits principaux obtenus par le processus industriel en question. De tels « co-produits » sont dans l'art antérieur peu ou non valorisés.

Par « biodégradable », on comprend que le film selon l'invention est un film qui peut être dégradé naturellement par des organismes vivants tels que par des bactéries. Cette dégradation pourra passer par une étape de fragmentation du film.

Les films selon la présente invention sont d'origine naturelle dans la mesure où les co-produits entrant dans leur composition n'ont subit aucun traitement chimique et que les éventuels autres produits que lesdits co-produits entrant aussi dans cette composition sont également des produits d'origine végétale ou animale n'ayant subi aucun traitement chimique.

L'utilisation de co-produits et non de produits principaux tels que les graines et les farines, pour la fabrication du film selon l'invention, est tout à fait acceptable du point de vue éthique. Elle est en outre en accord avec les exigences nouvelles de développement durable.

Préférentiellement, le film est réalisé à partir d'au moins un co-produit issu d'un processus de traitement de graines de soja.

On connaît dans l'art antérieur différents processus industriels de traitement des graines de soja conduisant à divers co-produits.

Ainsi, le «lait de soja », qui en fait est un jus, également connu de l'homme de l'art français sous le terme japonais « tonyu », est obtenu par le pressurage des graines de soja.

Selon le processus industriel classique de traitement de telles graines de soja pour l'obtention de « lait de soja », celles-ci sont d'abord dépelliculées afin de les débarrasser de leurs coques.

Les fèves ainsi obtenues sont réhydratées avec de l'eau (le pourcentage pondéral de fèves de soja utilisé par rapport à l'eau étant généralement d'environ 10%) puis les graines sont broyées et le broyat obtenu est centrifugé pour donner d'une part le « lait de soja » et d'autre part une pulpe résiduelle également appelée « okara ».

Ce « lait de soja » peut subir une ultrafiltration sur membranes à l'issue de laquelle on récupère d'une part un rétentat et d'autre part un perméat.

Le processus industriel du traitement des graines de soja pour l'obtention du « lait de soja » conduit donc à deux sous-produits, à savoir les coques et l'okara. Lorsque ce « lait de soja » est ultrafiltré pour la fabrication de yaourts, un co-produit supplémentaire, à savoir le perméat est obtenu.

Il est également connu d'extraire l'huile et les protéines du soja à partir des graines de soja. Les coques de soja constituent ce qui reste de la graine après une telle extraction. Ces coques constituent donc un co-produit de tels processus d'extraction des graines de soja.

Selon l'art antérieur, les co-produits issus de traitement industriel des graines de soja, à savoir les coques, l'okara et le perméat sont peu ou pas valorisés.

Selon l'invention, le ou les co-produits issus d'un processus de traitement de graines de soja pour la fabrication d'un film naturel biodégradable sont préférentiellement choisis dans le groupe constitué par l'okara et le perméat de filtration de « lait de soja ».

On notera que l'okara pourra être utilisé sous forme d'okara sec, c'est-à-dire contenant au moins 95 % en masse d'extrait sec ou sous forme d'okara humide, c'est-à-dire contenant entre 20 % et 25 % en masse d'extrait sec.

Les films naturels biodégradables selon l'invention sont susceptibles de présenter des propriétés au moins égales à celles des films de l'art antérieur, notamment en termes de résistance mécanique et de non-toxicité.

De manière totalement inattendue, les inventeurs ont mis en évidence qu'en outre, l'utilisation de co-produits issus d'un processus industriel de traitement des graines de soja permet d'obtenir des films biodégradables ayant une vitesse et un rendement de dégradation améliorée par rapport aux films de l'art antérieur.

Selon un aspect particulier, le film naturel et biodégradable selon l'invention est un film alimentaire.

Par film alimentaire, on comprend tout film agro-alimentaire destiné :
- à la protection de produits tels que le chocolat, les gâteaux secs, les oeufs, les fruits, les légumes, les produits d'épicerie sèche ... ;
- à la composition de matériaux pour supports alimentaires tels que canettes, bouteilles, barquettes, pots, ... ;
- à la réalisation de films couvre-sol ou paillage. Après la récolte des fruits et/ou légumes, le film est mélangé avec de la terre et sert alors d'engrais.

Selon un autre aspect, le film naturel et biodégradable selon l'invention est un film d'emballage.

Selon encore un autre aspect, le film selon l'invention est un excipient d'une forme galénique solide pour l'application d'un principe actif.

Par forme galénique solide, outre le film lui-même, on comprend toute forme individuelle sous laquelle sont mis le ou les principe(s) actif(s) et le ou les excipient(s) pour constituer un médicament. Elle correspond à l'aspect physique du médicament tel qu'il est utilisé par le patient. On pourra notamment citer : les bâtons, les comprimés, les capsules à enveloppe molle, les dragées, les gélules et les pilules. Actuellement, les capsules et les gélules utilisent comme excipient majoritaire soit de la gélatine (protéine animale) soit de la cellulose (polysaccharide d'origine végétale). Pour l'obtention de ces formes galéniques, le film selon l'invention sera tout simplement enroulé sur lui-même.

Selon encore un autre aspect particulièrement intéressant, le film naturel et biodégradable selon l'invention est un film à usage pharmaceutique ou médical pour l'application topique d'au moins un principe actif.

Selon une variante intéressante, le film selon l'invention est à usage gynécologique.

Préférentiellement, le film selon l'invention comprend dans sa composition au moins un agent filmogène.

Avantageusement, ledit agent filmogène est choisi dans le groupe constitué par les caséinates et des fractions protéiques issues de procédé de fabrication de produits laitiers. Par « caséinates » on entend : caséinate de sodium, caséinate de potassium, caséinate de calcium et caséinate de magnésium principalement. Ces produits d'origine naturelle sont en effet facilement disponibles.

Selon une variante, le film selon l'invention comprend dans sa composition au moins un agent plastifiant.

Préférentiellement ledit agent plastifiant est le glycérol. Ce composé présente l'avantage d'être biocompatible.

Avantageusement, le film selon l'invention, à tout le moins lorsqu'il sera à usage pharmaceutique ou médical, comprend dans sa composition :
de 0 % à 40% en masse de perméat ;
de 0% à 20 % en masse d'okara;
de 5% à 60 % en masse de plastifiant ;
de 20 % à 90 % en masse d'agent filmogène ;
de 0 à 20 % en masse d'eau.

L'invention porte également sur un produit d'administration d'au moins un principe actif caractérisé en ce qu'il comprend au moins un film à usage pharmaceutique ou médical tel que décrit ci-dessus et au moins un principe actif associé audit film. L'association dudit au moins un principe actif au film pour être mise en oeuvre par différente technique : dépôt en surface, imprégnation, incorporation au mélange initial. Un tel film peut être appliqué sur une muqueuse.

Selon une variante, ledit film auquel est associé ledit au moins un principe actif est destiné à être appliqué sur les lèvres ou à l'intérieur de la bouche.

Selon une autre variante, ledit film auquel est associé ledit au moins un principe actif est destiné à être appliqué sur la vulve ou dans le vagin.

Les conséquences du vieillissement post-ménopausique de l'appareil uro-génital ne s'expriment, le plus souvent, que plusieurs années après le début de la ménopause, raison pour laquelle, les pathologies qui en découlent sont assez mal connues du public et relativement mal prises en charge par le corps médical.

La carence oestrogénique locale entraîne des modifications physico-chimiques bien connues des tissus musculaires du périnée, épithéliaux du vagin, de l'urètre et de la vessie, aboutissant à des pathologies invalidantes souvent associées :
- sécheresse et atrophie vaginales pouvant entraîner des douleurs et des infections ;
- modifications de la flore bactérienne vaginale conduisant à des infections urinaires à répétition (14 % des femmes étant concernées vers l'âge de 65 ans, 25 % vers l'âge de 85 ans) ;
- incontinence urinaire à l'effort ou par hyperactivité vésicale (fréquence et impériosités mictionnelles) ;
- prolapsus, ....

En France, on estime que 5 millions de femmes souffrent d'incontinence urinaire (200 millions dans le monde). 15 à 20 % des femmes après 60 ans présentent une incontinence urinaire soit à l'effort, soit par hyperactivité vésicale ou mixtes.

Le traitement par un oestrogène de substitution, local, permet de prévenir, d'améliorer voire de guérir la majeur partie des femmes concernées, soit seul en traitement d'entretien continu, soit associé à d'autres principes actifs tel que par exemple des principes actifs anti-cholinergiques.

Plusieurs oestrogènes de substitution sont connus et présentent la même efficacité de traitement. On peut citer, notamment, l'oestriol, l'oestradiol, le promestriène. Cependant de nombreuses interrogations se posent quant à la relation entre l'utilisation de ces oestrogènes de substitution après la ménopause et le développement d'un cancer du sein. Ces interrogations freinent la prescription d'oestrogènes en prévention ou en traitement du vieillissement post-ménopausique de l'appareil uro-génital. Ainsi, l'utilisation du promestriène (Colpotrophine®) par voie vaginale est déconseillée par le fabriquant pour les patientes ayant des antécédents de cancer du sein. En revanche, les phytooestrogènes ne sont pas contre-indiqués. Ils auraient au contraire un effet préventif dans le cancer du sein. Aucune étude n'a actuellement pu démontrer de manière certaine de lien entre l'utilisation de phytooestrogènes de substitution et le développement du cancer du sein.

L'utilisation par voie orale des oestrogènes de substitution n'a pas d'effet probant sur les différentes formes d'incontinence, au contraire, certaines études montrent qu'une telle utilisation peut les aggraver.

Il existe actuellement des crèmes vaginales. Ce mode d'administration local permet de mieux contrôler et cibler les effets des oestrogènes mais sont difficiles à appliquer.

L'invention propose donc un produit d'administration d'au moins un principe actif incluant un film réalisé à partir d'au moins un co-produit issus d'un procédé de traitement de graines de soja destiné à être appliqué sur la vulve ou dans le vagin dans lequel ledit au moins un principe actif est un oestrogène ou un pseudo-oestrogène.

Avantageusement, ledit principe actif est contenu dans un extrait de graines de soja. Un tel extrait de graines de soja inclut préférentiellement un mélange de daidzine et de génistine. Ces composés sont des isoflavones et constituent des pseudo-oestrogènes d'origine végétale ou phytooestrogènes.

Selon une variante, le produit d'administration incluant un film destiné à être appliqué sur la vulve ou dans le vagin comprend un deuxième principe qui préférentiellement est un probiotique.

Comme il a été expliqué précédemment, à la ménopause, la carence oestrogénique induit des troubles trophiques responsables de manifestations pathologiques. La flore vaginale se modifie, les lactobacilles diminuent de 50 %, les anaérobies prédominent, en raison de processus de peroxydation induits par des infections liées à la diminution de la flore de Doderlein. Cette carence oestrogénique entraîne également une augmentation de l'adhérence bactérienne, en outre favorisée par l'élévation du pH, et qui est à l'origine de nombreux troubles, nycturie, pollakiurie, incontinence, infections urinaires, ....

Il apparaît ainsi qu'un apport direct de probiotiques dans le vagin peut contribuer à rétablir l'équilibre bactérien de la flore vaginale et ainsi éviter le développement de pathologies.

Selon une variante, un produit d'administration selon l'invention comprend deux compartiments distincts operculés par au moins un opercule, l'un desdits deux compartiments contenant ledit film associé audit premier principe actif et l'autre desdits deux compartiments contenant au moins un second principe actif, lesdits deux compartiments étant destinés à être repliés l'un sur l'autre après enlèvement dudit opercule, de façon telle que le second principe actif soit déposé sur le film.

L'application du film pourra être réalisée avec les doigts ou à l'aide d'un applicateur vaginal tel que les applicateurs décrits notamment dans les demandes de brevet FR 2 872 702 A3, US 2003/229328 A1, US 2005/171463 A1, US 2005/197615 A1, US 2005/273039 A1 et les brevets GB 2 412 321 B1 et US 7 591 808 B2. Lorsqu'un applicateur sera utilisé, le film aura avantageusement une forme cylindrique ou en doigt de gant, de telles formes constituent des supports performants pour l'apport des oestrogènes ainsi que des autres principes actifs, notamment des probiotiques. Les films de forme cylindrique ou en doigt de gant sont placés dans un applicateur afin qu'ils puissent être plus facilement délivrés dans la zone d'intérêt. Préférentiellement, les applicateurs utilisés seront à usage unique.

L'invention, ainsi que les avantages qu'elle procure, seront plus facilement compris grâce à la description qui va suivre d'exemples non limitatifs de réalisation de films et de produits d'administration de principes actifs incluant de tels films selon celle-ci, faite en référence aux dessins dans lesquels :
- la figure 1 est un organigramme relatif au protocole d'évaluation de la diffusion d'isoflavones à partir de films selon l'invention vers une matrice simulant la muqueuse vaginale ;
- la figure 2 représente deux graphes traduisant la diffusion d'isoflavones à partir de films selon l'invention dans la matrice ;
- la figure 3 représente deux graphes traduisant l'influence du taux d'agent filmogène des films selon l'invention dans la diffusion d'isoflavones dans la matrice ;
- la figure 4 représente deux graphes traduisant l'influence du taux de revêtement hydrophobe des films selon l'invention dans la diffusion d'isoflavones dans la matrice ;
- la figure 5 représente deux graphes traduisant l'influence du taux d'agent plastifiant selon l'invention dans la diffusion d'isoflavones dans la matrice ;
- la figure 6 représente un mode de réalisation d'un produit d'administration incluant un film biodégradable imprégné de principes actifs à usage pharmaceutique selon l'invention.

### Exemples de films biodégradables selon l'invention

Différents films biodégradables selon la présente invention ont été réalisés avec le procédé de moulage (« casting ») décrit ci-après.

Pour chaque formulation de film, détaillée dans le tableau 1 ci-dessous, différentes fractions de co-produits issus du traitement de graine de soja en vue de l'obtention de produits finis à base de soja, la fraction perméat et la fraction Okara, ont été mélangées avec une fraction d'agent filmogène et une fraction de plastifiant.

Dans la cadre du présent mode de réalisation, ce mélange a été versé sur un support en polystyrène (PS) en forme de cuve rectangulaire. Après évaporation de l'excès d'eau, les films obtenus ont été démoulés puis enfermés dans un récipient dans lequel l'atmosphère est maintenue à 53% d'humidité relative grâce à une solution saturée de nitrate de magnésium (Mg(NO₃)₂). Les films ont ensuite été découpés en éprouvettes aptes à subir différents tests mécaniques.

On notera que pour une mise en oeuvre industrielle, il pourra être prévu d'effectuer le mélange grâce à un mélangeur interne industriel, tel qu'un mélangeur portant la dénomination commerciale "HAAKE Polydrive". Un tel matériel, disponible dans le commerce, comprend une chambre de mélange dans laquelle tournent en sens contraires deux rotors en forme d'hélice. Ensuite le mélange pourra être thermopressé dans une presse hydraulique chauffante telle que celle disponible dans le commerce portant la dénomination commerciale « CARVER3860-416 ».

**Tableau 1**

| Formulation n° | Perméat % pondéral | Okara % pondéral | Agent Filmogène (% pondéral) | Total T (Perméat + Okara + Agent Filmogène) | Plastifiant (% pondéral par rapport au Total T) |
|---|---|---|---|---|---|
| 0 | 60 | 0 | 40 | 100 | 10 |
| 1 | 30 | 30 | 40 | 100 | 20 |
| 2 | 20 | 20 | 60 | 100 | 20 |
| 3 | 20 | 30 | 50 | 100 | 20 |
| 4 | 30 | 20 | 50 | 100 | 20 |
| 5 | 30 | 30 | 40 | 100 | 20 |
| 6 | 30 | 30 | 40 | 100 | 30 |

Dans ces formulations l'agent filmogène est du caséinate de sodium (NaCas), à l'exception de la formulation 3 où il s'agit de poudre de concentré de babeurre (PCB), et le plastifiant du glycérol (gly).

Chaque fraction des formulations utilisées est caractérisée dans le Tableau 2 ci-après.

**Tableau 2**

| **Fraction** | **Humidité (% en masse)** | **Protéines (% en masse)** | **Lipides (% en masse)** | **Minéraux (% en masse)** | **Sucres (% en masse)** | **Autres (% en masse)** |
|---|---|---|---|---|---|---|
| Okara | 4 | 30 | 15 | 4 | 0,5 | 46,5 (fibres) |
| Perméat | 4 | 10 | 0 | 9 | 45 | 32 (cendres) |
| NaCas | 6 | 90 | 0,5 | 3,5 | - | - |

5 éprouvettes du film répondant à la composition 0 présentant une largeur de 10 cm, une largeur de 1,5 cm et une épaisseur d'environ 200 µm ont été découpées afin de mesurer leurs propriétés mécaniques par des essais de traction. La machine de traction utilisée est une *Synergie RT 1000* de chez MTS avec un capteur de force de 250 N. Les éprouvettes ont toutes été conservées à un taux d'humidité de 53 % avant de réaliser les tests de traction.

Les résultats exposés au tableau 3 suivant sont des moyennes obtenues sur les 5 éprouvettes :

**Tableau 3**

| **Propriété Mécanique** | **Résultats (valeur moyenne)** |
|---|---|
| Module Elastique E (MPa) | 55 |
| Contrainte à la rupture σᵣ(MPa) | 1,8 |
| Allongement à la rupture εᵣ (%) | 70 |

Ces tests démontrent que la fraction perméat permet de conférer aux films de bonnes propriétés mécaniques.

L'okara riche en fibres (60% dans le produit sec) confère au film un caractère rigide et cassant. Ceci est recherché dans les films pharmaceutiques pour permettre à ceux-ci de se désagréger.

### Exemples de produits d'administration de principes actifs selon la présente invention

Les films selon la présente invention répondant aux formulations 1 à 6 décrites ci-dessus ont été associés à un extrait obtenu à partir de graines de soja. L'extrait est incorporé directement au mélange à une teneur de 4,2 % en masse. Cet extrait présente la particularité d'être riche en isoflavones et notamment de présenter des teneurs importantes en daidzine et en génistine.

Ces isoflavones ont des propriétés pseudo-oestrogéniques et peuvent donc permettre de lutter contre les carences en oestrogènes apparaissant lors de la ménopause. Elles constituent à ce titre un principe actif. Elles sont présentes sous deux formes dans l'extrait de graines de soja en question, à savoir la forme glycosylée (daidzine et génistine) et la forme aglycone (daidzéine et génistéine). Le tableau 4 présente les structures de ces composés.

**Tableau 4**

| | | |
|---|---|---|
| Forme glycosylée | 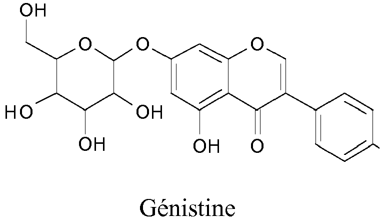 | 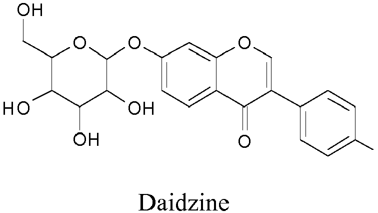 |
| Forme aglycone | 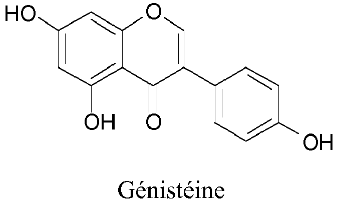 | 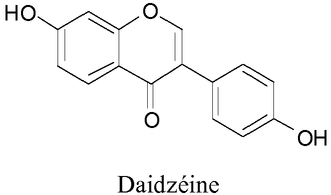 |

On notera que la forme aglycone est la seule à pouvoir passer dans la circulation sanguine et donc à pouvoir être assimilée directement par l'organisme. Cependant, la forme glycosylée peut être hydrolysée en forme aglycone par la flore vaginale. Ceci est très intéressant pour une diffusion progressive du principe actif. La composition précise de l'extrait de graines de soja en question est donnée dans le Tableau 5 ci-dessous.

**Tableau 5**

| **Composant** | **Proportion pondérale** |
|---|---|
| Isoflavones | Min. 40% |
| dont : | |
| Daidzine | 17,5% |
| Génistine | 16,3% |
| Autres composants naturels du soja | 40-50% |
| dont : | |
| Protéines | Max. 12% |
| Corps gras | Max. 1% |
| Métaux lourds | Max. 5 ppm |
| Humidité | Max. 7% |

Les films associés à cet extrait riche en isoflavones forment des produits d'administration des principes actifs que cet extrait contient vers la muqueuse vaginale.

Les tests exposés ci-après démontrent l'utilisation possible de films selon l'invention pour la réalisation de produits d'administration de principes actifs par diffusion de ceux-ci du film vers la muqueuse vaginale.

Dans ces tests, aux fins de modéliser la muqueuse vaginale, une gélose présentant une concentration de 30 g/l d'Agar a été utilisée.

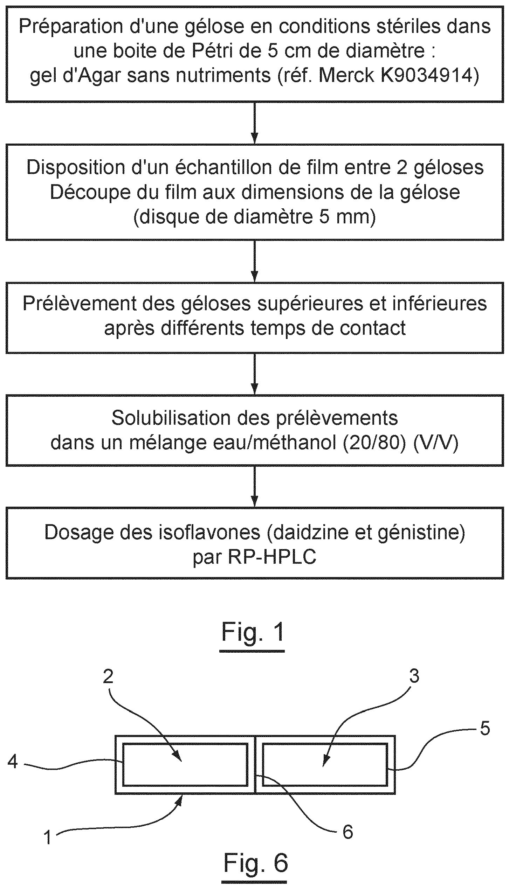

Le protocole général d'étude de la diffusion dans la matrice est donné à la figure 1.

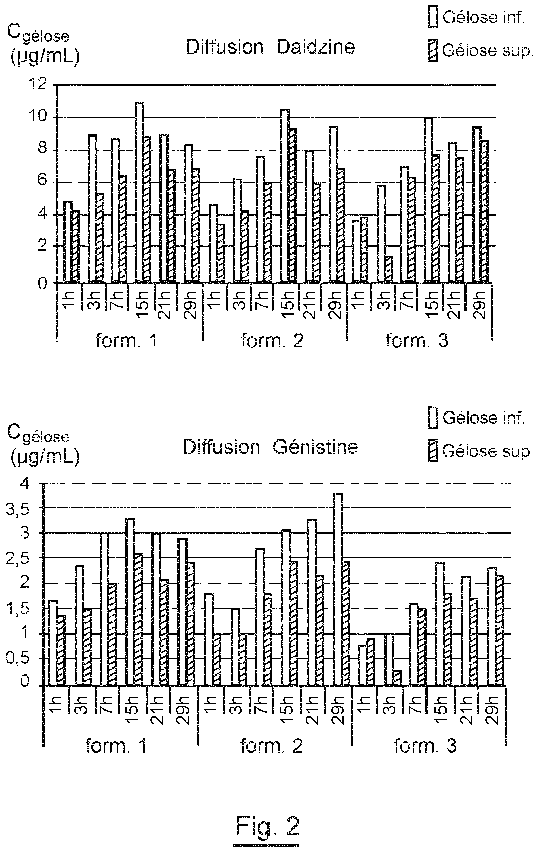

La diffusion de la daidzine et de la génistine dans la gélose inférieure et dans la gélose supérieure de la matrice a été étudiée sur les films répondant aux formulations 1,2 et 3 décrites ci-dessus. Les résultats de cette étude sont indiqués dans les deux graphes portés en figure 2.

Comme on peut le voir sur ces graphes, l'évolution de la concentration de la daidzine dans la gélose en fonction du temps est similaire à celle de la génistine.

De plus, la concentration de la daidzine dans la gélose à l'équilibre est d'environ 10 µg/mL alors que celle de la génistine n'est que de 3,5 µg/mL. La proportion de chacune des 2 isoflavones étant similaire dans le produit commercial concentré en isoflavones (17,5% pour la daidzine et 16,3% pour la génistine), ceci permet donc de mettre en évidence le fait que la daidzine diffuse plus facilement que la génistine au sein de la gélose.

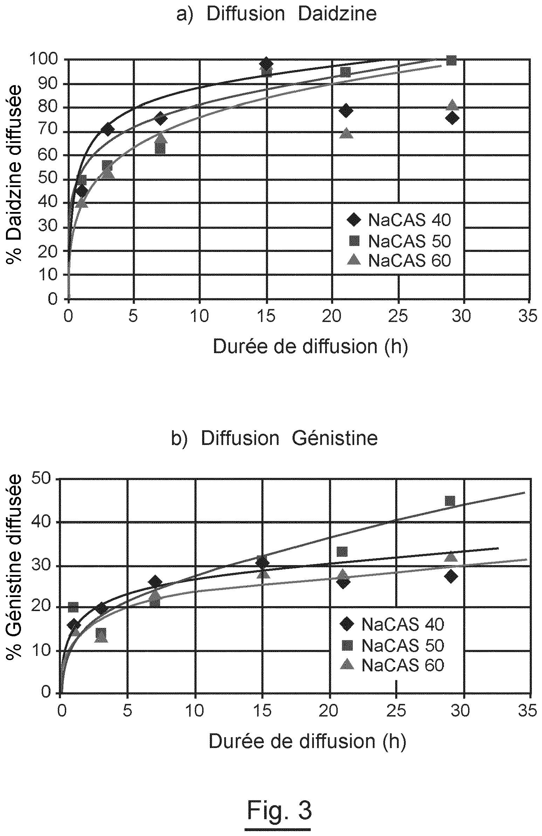

L'influence de la proportion pondérale en agent filmogène (caséinate de sodium (NaCAS)) dans les films selon l'invention répondant aux formulations 5, 2 et 4 décrits ci-dessus sur la diffusion de la daidzine (a) et de la génistine (b) a également été étudiée. Les résultats de cette étude sont indiqués dans les deux graphes portés en figure 3 dans lesquels la concentration en isoflavones diffusée a été ramenée en pourcentage par rapport au cas où tout le principe actif aurait diffusé.

L'interprétation de ces deux graphes permet de conclure que le profil de diffusion des deux isoflavones dans les différents films selon l'invention est similaire. Il apparaît toutefois que plus la proportion en agent filmogène augmente dans la formulation et plus la cinétique de diffusion diminue. Ce résultat est logique dans la mesure où une fraction protéique plus grande implique un caractère cohésif du film plus marqué rendant ainsi la diffusion probablement plus difficile. Ces graphes indiquent également que la vitesse de diffusion reste relativement élevée, notamment dans le cas de la daidzine, où plus de 90% de l'isoflavone a diffusé après 24h de temps de contact. Comme évoqué au paragraphe précédent, la génistine diffuse plus lentement. Ainsi, après 24h, seulement 30 à 45% de l'isoflavone a diffusé vers la gélose selon la proportion en caséinate de sodium dans la formulation. Cependant, après 30h, l'état d'équilibre n'est toujours pas atteint puisque la courbe continue à augmenter. Ce comportement peut être intéressant pour une libération plus progressive de cet actif.

L'influence d'un revêtement hydrophobe en surface du film a également été étudiée.

L'agent hydrophobe retenu pour cette étude est la lécithine de soja. Une suspension de ce composé a ainsi été élaborée à partir de 5 g de poudre de lécithine de soja mélangée à 10 mL d'eau ultra pure. Les films répondant aux formulations 1 et 2 ont ensuite été enduits de cette suspension (sur les faces supérieure et inférieure) puis disposés sur la gélose afin d'effectuer des tests de migration.

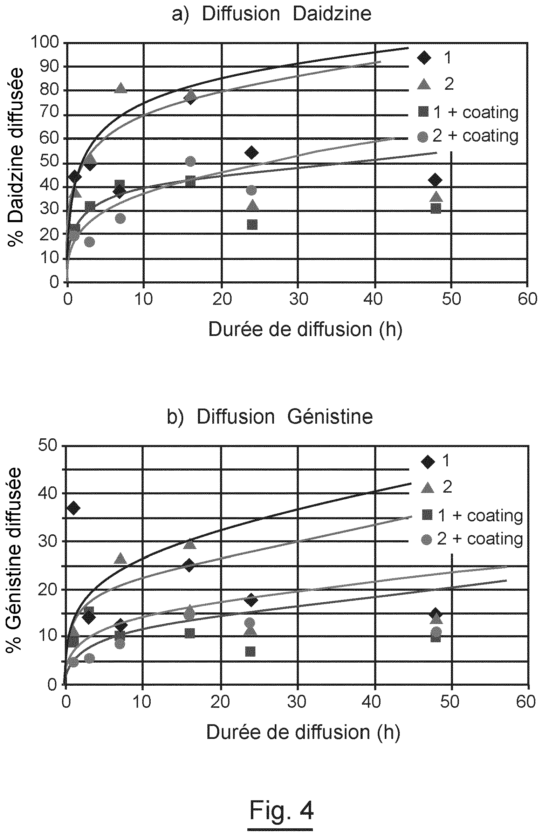

Les résultats de cette étude sont indiqués dans les deux graphes portés en figure 4 qui indiquent les profils de diffusion des isoflavones avec et sans revêtement hydrophobe.

Le profil de diffusion des deux isoflavones est ici encore similaire. On remarque également que la cinétique de diffusion de la génistine est toujours plus lente. En effet, après 48h de contact, environ 90% de la daidzine a diffusé dans la gélose pour les deux formulations sans revêtement alors que seulement 40% de la génistine a diffusé après le même temps de contact (respectivement 40% et 20% pour la daidzine et la génistine dans le cas des formulations avec revêtement de lécithine de soja).

La présence d'un revêtement de lécithine de soja à la surface du film permet de diminuer significativement la quantité de principe actif diffusant vers la gélose, et ce pour la daidzine comme pour la génistine. En effet, les quantités de daidzine et de génistine ayant diffusées dans la gélose après 48h passent respectivement de 90% à 40% et de 40% à 20%. Ceci peut avoir deux origines. D'une part, le fait de rajouter une couche supplémentaire augmente le chemin à parcourir et de ce fait la durée de diffusion. D'autre part, le caractère hydrophobe de cette couche engendre des interactions répulsives avec les isoflavones fortement polaires diminuant ainsi leur vitesse de migration vers la gélose. En outre, cette couche hydrophobe limite l'infiltration de molécules d'eau au sein du film et donc l'extraction des isoflavones par l'eau.

Il est également à noter que la comparaison des deux formulations étudiées ici permet d'aboutir à la même conclusion qu'au paragraphe précédent, à savoir qu'une plus grande fraction d'agent filmogène (NaCAS) dans la formulation, générant un matériau plus cohésif, permet de diminuer la vitesse de diffusion pour les films sans revêtement hydrophobe. En revanche, pour les formulations avec un revêtement hydrophobe, ce constat est moins marqué, vraisemblablement à cause des interactions répulsives évoquées ci-dessus.

Enfin, l'influence de la proportion pondérale en agent plastifiant (glycérol (gly)) dans les films selon l'invention sur la diffusion de la daidzine (a) et de la génistine (b) a également été étudiée.

Deux taux de plastification ont été examinés, correspondant aux formulations 4 et 5 des films selon l'invention décrites ci-dessus.

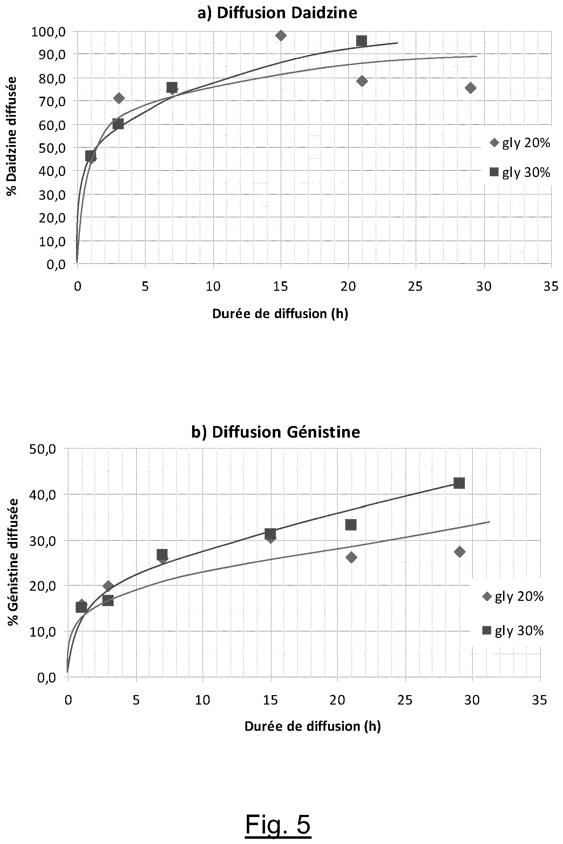

Les résultats de cette étude sont indiqués dans les deux graphes portés en figure 5.

L'interprétation de ces graphes indique que la génistine diffuse toujours plus lentement que la daidzine, 40% de la totalité de la génistine présente initialement dans le film ayant migré après 30h de contact contre 90% à 100% pour la daidzine. Un taux de plastification plus important engendre une légère augmentation de la vitesse de diffusion des principes actifs ainsi que de la quantité diffusée. En effet, pour un taux de glycérol de 30% en masse, quasiment 100% de la daidzine et 45% de la génistine ont diffusé après 30h contre environ 90% et 35% pour la daidzine et la génistine respectivement pour un taux de plastification de 20%. Ce résultat est logique dans le sens où un taux de plastification plus important implique une plus grande mobilité des chaînes, ce qui favorise la diffusion des isoflavones.

On décrit ci-après un autre mode de réalisation d'un produit d'administration de principes actifs incluant un film biodégradable selon la présente invention.

Dans ce mode de réalisation, le produit 1 d'administration de phytooestrogènes et de probiotiques selon la présente invention comprend deux compartiments 2, 3 distincts operculés par un blister transparent. Ces deux compartiments 2,3 sont en matériaux plastiques de forme rectangulaire et de faible épaisseur.

Le premier compartiment 2 contient, sous atmosphère neutre, un film pharmaceutique 4 selon l'invention réalisé à partir de co-produit issus d'un processus industriel de traitement de graine de soja et imprégné par un premier principe actif tel que décrit ci-dessus.

Le second compartiment 3 contient, sous atmosphère neutre, un second principe actif 5 sous forme d'un lyophilisat ayant l'aspect d'une poudre.

Dans le mode de réalisation présenté, le premier principe actif est constitué par des phytooestrogènes sous forme aglycone constitués par des isoflavones issus de graines de soja, sous forme glycosylée ou encore sous forme aglycone à une teneur comprise entre 10 et 100 mg/dose/jour, préférentiellement à une teneur de 70 mg/dose/jour.

Le second principe actif est quant à lui constitué par des probiotiques lyophilisés, à savoir des bactéries *Lactobacillus acidophilus* à une teneur de 20 mg/dose/jour (10⁹ CFU/dose/jour).

Dans le mode de réalisation présenté, lors de l'utilisation du produit d'administration, le blister couvrant les deux compartiments 2 et 3 est retiré.

Le compartiment 3 est ensuite replié sur le compartiment 2 selon une rainure de pliage 6 ce qui provoque l'adhésion des probiotiques sur le film pharmaceutique déjà imprégné par les phytooestrogènes. Le film imprégné de phytooestrogènes et de probiotiques est ensuite retiré du compartiment qu'il occupe.

Ce film imprégné des deux principes actifs, qui se présente alors sous la forme d'un feuillet lubrifié, peut alors être introduit dans le vagin.

Le film se désagrège à l'intérieur du vagin, libérant de manière ciblée et efficace ces principes actifs.

Un tel film peut être utilisé chez la femme ménopausée pour des soins post ou pré opératoires ou pour le traitement local de troubles trophiques du vagin.

## Revendications

1. Film biodégradable **caractérisé en ce qu'**il est réalisé à partir d'au moins un co-produit issu d'un processus de traitement de graine de soja visant à l'obtention de produits finis à base de soja par ultrafiltration, ledit co-produit étant choisi dans le groupe comprenant l'okara et le perméat.

2. Film selon la revendication 1 **caractérisé en ce qu'**il constitue un film alimentaire.

3. Film selon l'une quelconque la revendication 1 **caractérisé en ce qu'**il constitue un film d'emballage.

4. Film selon la revendication 1 **caractérisé en ce qu'**il constitue un excipient d'une forme galénique solide pour l'application d'un principe actif.

5. Film selon la revendication 4 **caractérisé en ce qu'**il constitue un film à usage pharmaceutique pour l'application topique d'au moins un principe actif.

6. Film selon la revendication 5 **caractérisé en ce qu'**il est à usage gynécologique.

7. Film selon l'une quelconque des revendications 1 à 6 **caractérisé en ce qu'**il comprend au moins un agent filmogène.

8. Film selon la revendication 7 **caractérisé en ce que** ledit agent filmogène est choisi dans le groupe constitué par les caséinates et des fractions protéiques issues de procédé de fabrication de produits laitiers.

9. Film selon l'une quelconque des revendications 1 à 8 **caractérisé en ce qu'**il comprend au moins un agent plastifiant.

10. Film selon la revendication 9 **caractérisé en ce que** ledit agent plastifiant est le glycérol.

11. Film selon les revendications 1, et 7 ou 8, et 9 ou 10 **caractérisé en ce qu'**il comprend :
de 0 % à 40% en masse de perméat ;
de 0% à 20 % en masse d'okara;
de 5% à 60 % en masse de plastifiant ;
de 20 % à 90 % en masse d'agent filmogène ;
de 0 à 20 % en masse d'eau.

12. Produit d'administration d'au moins un principe actif **caractérisé en ce qu'**il comprend au moins un film pharmaceutique selon l'une quelconque des revendications 5 à 11 et au moins un principe actif associé audit film.

13. Produit selon la revendication 12 **caractérisé en ce que** ledit film auquel est associé ledit au moins un principe actif est destiné à être appliqué sur une muqueuse.

14. Produit selon la revendication 13 **caractérisé en ce que** ledit film auquel est associé ledit au moins un principe actif est destiné à être appliqué sur les lèvres ou à l'intérieur de la bouche.

15. Produit selon la revendication 13 caractérisé en ce ledit film auquel est associé ledit au moins un principe actif est destiné à être appliqué sur la vulve ou dans le vagin.

16. Produit selon la revendication 15 **caractérisé en ce que** ledit au moins un principe actif est un oestrogène ou un pseudo-oestrogène.

17. Produit selon la revendication 16 **caractérisé en ce que** ledit au moins un principe actif est contenu dans un extrait de graines de soja.

18. Produit selon la revendication 17 **caractérisé en ce que** ledit au moins un principe actif inclut un mélange de daidzine et de génistine.

19. Produit selon l'une quelconque des revendications 12 à 18 **caractérisé en ce qu'**il inclut au moins un deuxième principe actif.

20. Produit selon la revendication 19 **caractérisé en ce que** ledit deuxième principe est un probiotique.

21. Produit selon l'une quelconque des revendications 19 ou 20 **caractérisé en ce qu'**il comprend deux compartiments distincts (2,3) operculés par au moins un opercule, l'un (2) desdits deux compartiments contenant ledit film (4) associé audit premier principe actif et l'autre (3) desdits deux compartiments contenant au moins un second principe actif (5), lesdits deux compartiments (2,3) étant destinés à être repliés l'un sur l'autre après enlèvement dudit opercule, de façon telle que le second principe actif (5) soit déposé sur ledit film (4).

## Patentansprüche

1. Biologisch abbaubarer Film, **dadurch gekennzeichnet, dass** er aus mindestens einem Coprodukt hergestellt wird, das aus einem Sojabohnenbehandlungsverfahren zur Gewinnung von Endprodukten auf der Basis von Soja durch Ultrafiltration stammt, wobei das Coprodukt aus der Gruppe ausgewählt ist, die aus Okara und dem Permeat besteht.

2. Film nach Anspruch 1, **dadurch gekennzeichnet, dass** er eine Klarsichtfolie darstellt.

3. Film nach einem von Anspruch 1, **dadurch gekennzeichnet, dass** er eine Verpackungsfolie darstellt.

4. Film nach Anspruch 1, **dadurch gekennzeichnet, dass** er einen Excipienten einer festen Darreichungsform für die Applikation eines Wirkstoffs darstellt.

5. Film nach Anspruch 4, **dadurch gekennzeichnet, dass** er einen Film zur pharmazeutischen Verwendung für die topische Applikation mindestens eines Wirkstoffs darstellt.

6. Film nach Anspruch 5, **dadurch gekennzeichnet, dass** er zur gynäkologischen Verwendung dient.

7. Film nach einem der Ansprüche 1 bis 6, **dadurch gekennzeichnet, dass** er mindestens ein filmbildendes Mittel umfasst.

8. Film nach Anspruch 7, **dadurch gekennzeichnet, dass** das filmbildende Mittel aus der Gruppe ausgewählt ist, bestehend aus Caseinaten und Proteinfraktionen, die aus einem Herstellungsverfahren von Milchprodukten stammen.

9. Film nach einem der Ansprüche 1 bis 8, **dadurch gekennzeichnet, dass** er mindestens einen Weichmacher umfasst.

10. Film nach Anspruch 9, **dadurch gekennzeichnet, dass** der Weichmacher Glycerin ist.

11. Film nach den Ansprüchen 1 und 7 oder 8 und 9 oder 10, **dadurch gekennzeichnet, dass** er Folgendes umfasst:
0 bis 40 Gew.-% Permeat;
0% bis 20 Gew.-% Okara;
5 bis 60 Gew.-% Weichmacher;
20 bis 90 Gew.-% filmbildendes Mittel;
0 bis 20 Gew.-% Wasser.

12. Produkt zur Verabreichung mindestens eines Wirkstoffs, **dadurch gekennzeichnet, dass** es mindestens einen pharmazeutischen Film nach einem der Ansprüche 5 bis 11 und mindestens einen mit dem Film assoziierten Wirkstoff umfasst.

13. Produkt nach Anspruch 12, **dadurch gekennzeichnet, dass** der Film, mit dem der mindestens eine Wirkstoff assoziiert ist, für die Applikation auf eine Schleimhaut bestimmt ist.

14. Produkt nach Anspruch 13, **dadurch gekennzeichnet, dass** der Film, mit dem der mindestens eine Wirkstoff assoziiert ist, für die Applikation auf die Lippen oder auf das Innere des Mundes bestimmt ist.

15. Produkt nach Anspruch 13, **dadurch gekennzeichnet, dass** der Film, mit dem der mindestens eine Wirkstoff assoziiert ist, für die Applikation auf die Vulva oder die Vagina bestimmt ist.

16. Produkt nach Anspruch 15, **dadurch gekennzeichnet, dass** der mindestens eine Wirkstoff ein Östrogen oder ein Pseudoöstrogen ist.

17. Produkt nach Anspruch 16, **dadurch gekennzeichnet, dass** der mindestens eine Wirkstoff in einem Sojabohnenextrakt enthalten ist.

18. Produkt nach Anspruch 17, **dadurch gekennzeichnet, dass** der mindestens eine Wirkstoff ein Gemisch von Daidzin und Genistin enthält.

19. Produkt nach einem der Ansprüche 12 bis 18, **dadurch gekennzeichnet, dass** es mindestens einen zweiten Wirkstoff enthält.

20. Produkt nach Anspruch 19, **dadurch gekennzeichnet, dass** der zweite Bestandteil ein Probiotikum ist.

21. Produkt nach einem der Ansprüche 19 oder 20, **dadurch gekennzeichnet, dass** es zwei getrennte Kompartimente (2, 3) umfasst, die mit mindestens einem Deckel verschlossen sind, wobei das eine (2) der beiden Kompartimente den Film (4) enthält, der mit dem ersten Wirkstoff assoziiert ist, und das andere (3) der beiden Kompartimente mindestens einen zweiten Wirkstoff (5) enthält, wobei die beiden Kompartimente (2, 3) dafür gestaltet sind, dass sie sich nach dem Abheben des Deckels aufeinander falten lassen, so dass der zweite Wirkstoff (5) auf den Film (4) aufgebracht wird.

## Claims

1. Biodegradable film **characterized in that** it is made out of at least one co-product derived from a process for treating soybeans aimed at obtaining soy-based finished products by ultrafiltration, said co-product being chosen from the group comprising okara and permeate.

2. Film according to claim 1 **characterized in that** it constitutes a food-grade film.

3. Film according to claim 1 **characterized in that** it constitutes a packaging film.

4. Film according to claim 1 **characterized in that** it constitutes an excipient with a solid galenic form for the application of an active ingredient.

5. Film according to claim 4 **characterized in that** it constitutes a film for pharmaceutical or medicinal use for the topical application of at least one active ingredient

6. Film according to claim 5 **characterized in that** it for gynecological use.

7. Film according to any one of the claims 1 to 6 **characterized in that** it comprises a film-forming agent.

8. Film according to claim 7 **characterized in that** said film-forming agent is chosen from the group constituted by caseinates and protein fractions derived from processes for manufacturing milk products.

9. Film according to any one of the claims 1 to 8 **characterized in that** it comprises at least one plasticizer.

10. Film according to claim 9 **characterized in that** said plasticizer is glycerol.

11. Film according to claims 1, and 7 or 8, and 9 or 10 **characterized in that** it comprises:
0% to 40% by mass of permeate;
0% to 20% by mass of okara;
5% to 60% by mass of plasticizer;
20% to 90% by mass of film-forming agent;
0% to 20% by mass of water.

12. Product for administering at least one active ingredient **characterized in that** it comprises at least one pharmaceutical film according to any one of the claims 5 to 11 and at least one active ingredient associated with said film.

13. Product according to claim 12 **characterized in that** said film with which at least one active ingredient is associated is intended to be applied to a mucous membrane.

14. Product according to claim 13 **characterized in that** said film with which said at least one active ingredient is associated is intended to be applied to the lips or to the inside of the mouth.

15. Product according to claim 13 **characterized in that** said film with which at least one active ingredient is associated is intended to be applied to the vulva or in the vagina.

16. Product according to claim 15 **characterized in that** said at least one active agent is an estrogen or a pseudo-estrogen.

17. Product according to claim 16 **characterized in that** said active ingredient is contained in an extract of soybeans.

18. Product according to claim 17 **characterized in that** said at least one active ingredient includes a mixture of daidzin and genistin.

19. Product according to any one of the claims 12 to 18 **characterized in that** it includes at least one second active principle.

20. Product according to claim 19 **characterized in that** said second active ingredient is a probiotic.

21. Product according to either of the claims 19 or 20 **characterized in that** it comprises two distinct compartments (2, 3) shut by at least one lid, one (2) of said two compartments containing said film (4) associated with said first active ingredient and the other (3) of said two compartments containing at least one second active ingredient (5), said two compartments (2, 3) being intended to be folded one on top of the other after removal of said lid so that the second active principle (5) is deposited on said film (4).
